# EUROPEAN PATENT APPLICATION

(11) **EP 1 543 827 A1**
(43) Date of publication of application: **22.06.2005**
(21) Application number: 02798387.3
(22) Date of filing: 25.09.2002
(51) Int. Cl.: A61K 9/107

(54) **EMULSION OF PERFLUORORGANIC COMPOUNDS EXHIBITING GAS-TRANSPORT PROPERTIES, A SURFACE-ACTIVE AGENT FOR SAID EMULSION AND METHOD FOR THE PRODUCTION THEREOF**

(71) Applicant: Obshestvo C Ogranichennoi Otvetstvennostiu Accent, Moscow 123056 (RU)
(72) Inventor: KALINICHENKO, Alla Nikolaevna, Moskovskaya obl., 141700 (RU); KAPTSOV, Vladimir Vasilievich, Moskovskaya obl., 142290 (RU); LAZAREV, Mikhail Ivanovich, Moskovskaya obl., 142290 (RU)
(74) Representative: Curtis, Philip Anthony
(86) International application number: PCT/RU2002/000431
(87) International publication number: WO 2004/047804

(57) **Abstract**

The group of inventions relates to medicine and colloid chemistry and can be used for producing gas-transport function blood substitutes, medicinal and cosmetic preparations and food additives. Said group of inventions makes it possible to obtain a high emulsion stability for long-term storage, in particular the storage at an above-zero temperature during an year, the emulsion stability to sterilisation (autoclaving), produce an emulsion with particle sizes less than 250 nm at an narrow dispersiveness thereof, increase the oxygen capacity and eliminate the toxicity of intravenously injected doses of 15-20 ml/kg. Said inventions are **characterised in that** tocopherol polyethylenglycol ester is used in the form of a surface-active substance for producing emulsion of perfluororganic compounds exhibiting gas-transport properties. The inventive emulsion contains a mixture of perfluororganic compounds and a surface-active substance which is embodied at least in the form of tocopherol polyethylenglycol ester or the mixture thereof with other nonionogenic surface-active substances. The inventive method for producing said emulsion consists in stabilising a mixture of perfluordecalin and perfluoro-n-methylcyclohexylpiperidine by an surface-active substance in the form of tocopherol polyethylenglycol ester or the mixture thereof with other nonionogenic surface-active substances.

## Description

### 1. Field of the Invention

The group of inventions relates to medicine and colloid chemistry and can be used for producing gas-transport function blood substitutes.

The proposed group of inventions can be used in medicine and medical industry for producing high-dispersion emulsions of perfluororganic compounds (PFOC), which are a basis of gas-transport blood substitutes and perfusion media for storing isolated donor organs at transplantation.

Besides, it can be used in pharmaceutical and food industries and in cosmetics, as well as in research practice, for preparation of high-dispersion PFOC-based emulsions serving as oxygen-carrying components of drugs and cosmetic ointments and creams.

### 2. Technical Background

PFOC emulsions are complex colloid systems comprising a dispersion medium (a solution of a surface-active substance (SAS) or a solution of several SASes in water) and a dispersion phase (a perfluororganic compound (PFOC) or a mixture of several PFOCs).

PFOC and SAS are basic components of the emulsion. However, the emulsion may also include other secondary components, e.g., salt solutions or thickening agents.

PFOC dissolve different gases physically well. Gases are much better soluble therein than in most other fluids. PFOC are chemically inert. Specific density thereof is twice higher than that of the water. PFOC are not dissolved in water, therefore to be used in medicine they have to be emulsified (atomized) and homogenized in the finest drops on special devices (homogenizers) in the presence of surface-active substances, which are concentrated on the surface of PFOC-water interface preventing the fusion of emulsion particles. The rate of particle fusion depends on the size thereof, the temperature and surface-active properties of selected SAS.

Although PFOC are chemically inert, they affect an organism when injected intravenously. Emulsion particles are captured and accumulated in different organs. Then they are gradually cleared out of the organism. The period of PFOC semi-clearance out of the organism is one of the characteristics of PFOC applicability for medical purposes. For blood substitutes, the period of PFOC semi-clearance must be short. It is 7 days for perfluordecalin (PFD) and 65 days for perfluoro-n-methylcyclohexylpiperidine (PMCP).

Non-toxic nonionogenic high-molecular compounds (NSAS), in particular, proxanols (foreign analogs are pluronics), are often used as SAS. Their amount in PFOC emulsions is as minimal as possible and determined only by necessity of satisfactory emulsification and homogenization of PFOC. Minimization of SAS amount also results from the fact that they affect the toxicity and reactogenicity of PFOC emulsion.

The mean diameter of emulsion particles depends both on the PFOC properties and on the ability of SAS to decrease surface tension on the PFOC-water interface. The less is surface tension, the easier proceeds emulsification and the less is the medium particle diameter of emulsion obtained.

PFOC emulsions with this or that SAS must have a long-term period of storage, and storage conditions must be simple. A characteristic of PFOC emulsion storage is the change (increase) in the mean diameter of emulsion particles during the storage.

The effect of PFOC emulsions on an animal or human organism is of diversified character:
1) Physicochemical effect of SAS and PFOC on the cells and immune system of an organism, blood cells and plasma, short- and long-term changes in metabolism thereof, etc.;
2) Effect of physicomechanical properties of emulsion, determined by particle diameter, dispersiveness, tendency to aggregation and, finally, emulsion viscosity, which is associated with the risk of capillary congestion and embolism development;
3) Effect of emulsion particles capture and accumulation by different organs;
4) Effect of physical evaporation of PFOC (the characteristic is PFOC vapor pressure), because these compounds are cleared out of an organism mainly through the lungs.

The safety of PFOC emulsion is determined by toxicity value thereof, which is assessed by index LD50. Index LD50 is a dose of injected emulsion in ml per kg of animal body weight, allowing the survival of 50% of animals. At the same time, different animals have different sensitivity and give different LD50 values. As a rule, LD50 is investigated in laboratory mice. Sometimes the toxicity is assessed by percentage of survived animals of the total amount of animals after intravenous injection of a certain dose.

The following parameters of PFOC emulsions determine the quality or prospects of application thereof:
1) Mean diameter of PFOC emulsion particles;
2) Distribution of emulsion particles by diameters;
3) Period of PFOC semi-clearance out of an organism;
4) Emulsion toxicity or LD50;
5) Oxygen capacity of emulsion;
6) Value of PFOC vapor pressure;
7) Sterilization of emulsion;
8) Possibility of long-term storage of emulsion;
9) SAS toxicity;
10) Reactogenicity of emulsion.

The above characteristics are compared in order to assess produced PFOC emulsions and to determine the advantage of one PFOC emulsion over another.

The main tasks at the synthesis of gas-transport PFOC emulsions are:
1) Decrease of the mean diameter of PFOC emulsion particles and constriction of distribution thereof by diameters;
2) Reliable sterilization of PFOC emulsions;
3) Reduction of toxicity of PFOC emulsions;
4) Enhancement of periods and simplification of conditions of storage of PFOC emulsions without significant changes in the medium particle diameter and broadening of particle distribution by diameters.

There are emulsions exhibiting gas-transport properties, which consist of different fluorocarbon compounds (patents US #5536753, A61K31/03, 17.06.1996, #5621144, C07C223/12, 15.04.1997, #5840767, A61K31/02, 24.11.1998). The above emulsions have a long-term stability and particle size of 0.05 to 0.25 nm and are quickly cleared out.

Disadvantages of the emulsions are high cost, necessity of special temperature regime during the storage, and possibility of degradation during sterilization.

A PFC emulsion is known (EP #0231070, A61K9/00, 31/02, 12.09.1998), which contains perfluoroctylbromide (PFOB) in an amount of 20 to 125 w% and is stabilized by phospholipids in the amount of 5 v%. The period of PFOB semi-clearance is 4 days. The mean diameter of PFOB particles is 277 nm, and 95% of particles have a diameter of less than 400 nm. It is stable for 18 months at +4°C. The index of stability D0/Dt is 1.4 (D0 is the mean particle diameter immediately after finishing the emulsion and Dt is the mean particle diameter after a certain period of storage). The emulsion has an oxygen capacity of 25 v% and low toxicity for rabbits.

Disadvantages of the emulsion are great mean particle diameter (more than 250 nm), insufficient stability, and high cost.

PFOC emulsions containing a mixture of two perfluororganic compounds are widely known. One PFOC is quickly cleared out of an organism but instable as emulsion. The other PFOC is cleared out during a longer time but helps stabilizing the emulsion at long-term storage. Such PFOC emulsions are stabilized either by nonionogenic SAS or by phospholipids.

The PFOC emulsion named "Perftoran" (patent RU #2070033, A61K9/10, 10.12.1996) is the closest to the proposed group of inventions. It contains two PFOC: perfluordecalyn (PFD) and perfluoro-n-methylcyclohexylpiperidine (PMCP), PFD:PMCP = 2:1. The PFOC mixture concentration in finished preparation is 10 v%.

The emulsion is stabilized by proxanol 268 in a concentration of 4 w%.

"Perftoran" also comprises a complex of salts and an oncotic component. The emulsion has a mean particle diameter of 70-150 nm. The Perftoran emulsion (PFD:PMCP = 2:1, 10 v%, proxanol 268 = 4 w%) has the following disadvantages:
- Low oxygen capacity;
- Possibility of long storage only in a frozen state;
- Low stability at storage without freezing;
- Impossibility of autoclaving.

### Summary of Inventions

An objective of the group of inventions is to produce emulsions of perfluororganic compounds that contain surface-active agents, possess no toxicity, and provide stability of said emulsions.

A technical result of the group of inventions is high emulsion stability at long-term storage, in particular, possibility of emulsion storage at above-zero temperatures for a year, emulsion stability at sterilization (autoclaving), production of an emulsion with particle size of less than 250 nm at narrow dispersivity thereof, enhanced oxygen capacity, and absence of toxicity at intravenous injection in a dose of 15-20 ml/kg.

The above mentioned technical result is achieved by using tocopherol polyethylenglycol ester as a surface-active substance for production of an emulsion of perfluororganic compounds with gas-transport properties.

An emulsion of perfluororganic compounds with gas-transport properties contains a mixture of perfluororganic compounds and a surface-active substance, which is at least tocopherol polyethylenglycol ester or mixture thereof with other nonionic surface-active substances.

The ratio of the amount of perfluororganic compounds to the amount of tocopherol polyethylenglycol ester is 20:1 to 3:1 by weight.

The ratio of the amount of perfluororganic compounds to the total amount of nonionogenic surface-active substances is 20:1 to 3:1 by weight.

The mixture of perfluororganic compounds is a mixture of perfluordecalin and perfluoro-n-methylcyclohexylpiperidine or other perfluorated compounds, with proxanol as one of surface-active agents.

Total concentration of the mixture of perfluororganic compounds in finished preparation is 5 v% to 30 v%.

In the method of producing an emulsion of perfluororganic compounds, where the mixture of perfluordecalin and perfluoro-n-methylcyclohexylpiperidine is stabilized by surface-active substance, tocopherol polyethylenglycol ester or mixture thereof with other nonionogenic surface-active substances is used a surface-active agent.

The above emulsion is produced at a pressure of 20-60 MPa with the number of homogenization cycles up to 30.

The emulsion is sterilized by autoclaving in the temperature range of 100 to 120°C.

### List of Chart Figures

Figure 1 shows the curves of a change in the mean particle diameter depending on the number of homogenization cycles for 3 different emulsions, where PFOC (20 v%) were stabilized by the following SAS: proxanol 268 (Pr268) in the concentration of 8 w%, SAS-3 in the concentration of 8 w%, and a mixture of SAS-3 and proxanol 268 in the ratio of 1:1 and total concentration of 8 w%. The X-axis gives the number of homogenization cycles and the Y-axis gives mean particle diameter in nm.

Figure 2 shows diagrams of the value of mean particle diameter of PFOC emulsions in different emulsions
a) Before autoclaving - front column;
b) Immediately after autoclaving - middle column;
c) 90 days after autoclaving - back column.

The X-axis gives the numbers of emulsions presented in the examples with the corresponding number. The Y-axis gives mean particle diameter in nm.

Figure 3 shows the curves of a change in the mean particle diameter of PFOC emulsions stabilized by different ratios of SAS-3 and proxanol 268 and with different total SAS concentrations depending on storage time.

The X-axis gives storage duration in days and the Y-axis gives mean particle diameter.

### Description of the Preferred Embodiments

Tocopherol polyethylenglycol ester used as an emulsifying and stabilizing component for producing an emulsion of perfluororganic compounds has been tentatively named as SAS-3.

Besides, the experiments have shown that SAS-3 has anti-oxidant properties that may be used for production of medicinal and cosmetic preparations, as well as food additives.

Emulsifiers used in the experiments were: SAS-3 and compositions thereof with proxanols (proxanol 268 and proxanol 168), glycide esters of fluorated alcohols-telomers, chitosan polyethylenglycol esters, etc. The weight ratio of PFOC to SAS-3 or to the total amount of nonionogenic SAS was 20:1 to 3:1.

Control PFOC emulsions were prepared on a high pressure homogenizer at 20 to 60 MPa in the cyclic mode of emulsion homogenization with the number of cycles N= 1 to N=30.

Mean particle diameter was measured by turbidity spectrum method using spectroturbidimetry data on a colorimeter KFK-2 MP in the light wavelength range of 340 nm to 750 nm. The mean diameter of emulsion particles was computed by the initial data of optical turbidity according to the technique described in literature by turbidity spectrum method [8-10].

All experiments on PFOC emulsion toxicity were carried out in two white non-pedigree laboratory mice of 20-35 g. The emulsion was injected intravenously into tail vein in a dose of 15-20 ml/kg. Survival of the mice was monitored during 3 days after the intravenous injection. Emulsion toxicity was judged by percentage of mice survival after the injected dose.

The addition of SAS-3 to PFOC emulsion made it possible to sterilize PFOC emulsion by autoclaving. Different temperature regimes of autoclaving were tested in the temperature range of 100 to 120°C.

Specific examples of optimal compositions of proposed PFOC emulsion with gas-transport properties are given below.

**Example 1.** PFOC emulsion with the concentration of PFD = 13.3 v% and concentration of PMCP = 6.67 v%, i.e., with the PFD:PMCP ratio of 2:1 and the total concentration of dispersion phase (PFOC) equal to 20 v%, was stabilized by proxanol 268 (Pr268) at a concentration of 6 w% and SAS-3 at a concentration of 2 w%, i.e., the ratio of SAS-3/Pr268 = 1:3 and total concentration of 8 w%. The emulsion was produced at a pressure of 40 MPa and homogenization cycle number N = 20.

The mean diameter of PFOC emulsion particles before the autoclaving was 85 nm. After sterilization (0.5 excess atmosphere for 20 min), the mean diameter of PFOC emulsion particles was 100 nm. After PFOC emulsion storage for 30 days at a temperature of +4-5°C, the mean diameter of PFOC emulsion particles was 147 nm.

Survival of the mice after intravenous injection in a dose of 15-20 ml/kg was 100%.

**Example 2**. PFOC emulsion with the concentration of PFD = 13.3 v% and concentration of PMCP = 6.67 v%, i.e., with the PFD:PMCP ratio of 2:1 and the total concentration of dispersion phase (PFOC) equal to 20 v%, was stabilized by proxanol 268 (Pr268) at a concentration of 4 w% and SAS-3 at a concentration of 4 w%, i.e., the ratio of SAS-3/Pr268 = 1:1 and total concentration of 8 w%. The emulsion was produced at a pressure of 40 MPa and homogenization cycle number N = 20.

The mean diameter of PFOC emulsion particles before the autoclaving was 90 nm. After sterilization (0.5 excess atmosphere for 30 min), the mean diameter of PFOC emulsion particles was 108 nm. After PFOC emulsion storage for 30 days at a temperature of +4-5°C, the mean diameter of PFOC emulsion particles was 143 nm.

Survival of the mice after intravenous injection in a dose of 15-20 ml/kg was 100%.

**Example 3**. PFOC emulsion with the concentration of PFD = 13.3 v% and concentration of PMCP = 6.67 v%, i.e., with the PFD:PMCP ratio of 2:1 and the total concentration of dispersion phase (PFOC) equal to 20 v%, was stabilized by proxanol 268 (Pr268) at a concentration of 3 w% and SAS-3 at a concentration of 1 w%, i.e., the ratio of SAS-3/Pr268 = 1:3 and total concentration of 4 w%. The emulsion was produced at a pressure of 40 MPa and homogenization cycle number N = 20.

The mean diameter of PFOC emulsion particles before the autoclaving was 97 nm. After sterilization (0.5 excess atmosphere for 45 min), the mean diameter of PFOC emulsion particles was 102 nm. After PFOC emulsion storage for 30 days at a temperature of +4-5°C, the mean diameter of PFOC emulsion particles was 134 nm.

Survival of the mice after intravenous injection in a dose of 15-20 ml/kg was 100%.

**Example 4**. PFOC emulsion with the concentration of PFD = 13.3 v% and concentration of PMCP = 6.67 v%, i.e., with the PFD:PMCP ratio of 2:1 and the total concentration of dispersion phase (PFOC) equal to 20 v%, was stabilized by proxanol 268 (Pr268) at a concentration of 2 w% and SAS-3 at a concentration of 2 w%, i.e., the ratio of SAS-3/Pr268 = 1:1 and total concentration of 4 w%. The emulsion was produced at a pressure of 40 MPa and homogenization cycle number N = 20.

The mean diameter of PFOC emulsion particles before the autoclaving was 122 nm. After sterilization (0.5 excess atmosphere for 60 min), the mean diameter of PFOC emulsion particles was 133 nm. After PFOC emulsion storage for 30 days at a temperature of +4-5°C, the mean diameter of PFOC emulsion particles was 154 nm.

Survival of the mice after intravenous injection in a dose of 15-20 ml/kg was 100%.

**Example 5.** PFOC emulsion with the concentration of PFD = 13.3 v% and concentration of PMCP = 6.67 v%, i.e., with the PFD:PMCP ratio of 2:1 and the total concentration of dispersion phase (PFOC) equal to 20 v%, was stabilized by proxanol 268 (Pr268) at a concentration of 3 w% and SAS-3 at a concentration of 1 w%, i.e., the ratio of SAS-3/Pr268 = 1:3 and total concentration of 4 w%. The emulsion was produced at a pressure of 60 MPa and homogenization cycle number N = 10.

The mean diameter of PFOC emulsion particles before the autoclaving was 92 nm. After sterilization (0.5 excess atmosphere for 60 min), the mean diameter of PFOC emulsion particles was 107 nm. After PFOC emulsion storage for 30 days at a temperature of +4-5°C, the mean diameter of PFOC emulsion particles was 134 nm. Survival of the mice after intravenous injection in a dose of 15-20 ml/kg was 100%.

**Example 6**. PFOC emulsion with the concentration of PFD = 13.3 v% and concentration of PMCP = 6.67 v%, i.e., with the PFD:PMCP ratio of 2:1 and the total concentration of dispersion phase (PFOC) equal to 20 v%, was stabilized by proxanol 268 (Pr268) at a concentration of 3 w% and SAS-3 at a concentration of 1 w%, i.e., the ratio of SAS-3/Pr268 = 1:3 and total concentration of 4 w%. The emulsion was produced at a pressure of 60 MPa and homogenization cycle number N = 30.

The mean diameter of PFOC emulsion particles before the autoclaving was 89 nm. After sterilization (0.5 excess atmosphere for 60 min), the mean diameter of PFOC emulsion particles was 97 nm. Survival of the mice after intravenous injection in a dose of 15-20 ml/kg was 100%.

**Example 7**. PFOC emulsion with the concentration of PFD = 13.3 v% and concentration of PMCP = 6.67 v%, i.e., with the PFD:PMCP ratio of 2:1 and the total concentration of dispersion phase (PFOC) equal to 20 v%, was stabilized by proxanol 268 (Pr268) at a concentration of 3 w% and SAS-3 at a concentration of 1 w%, i.e., the ratio of SAS-3/Pr268 = 1:3 and total concentration of 4 w%. The emulsion was produced at a pressure of 20 MPa and homogenization cycle number N = 10.

The mean diameter of PFOC emulsion particles before the autoclaving was 120 nm. After sterilization (0.5 excess atmosphere for 60 min), the mean diameter of PFOC emulsion particles was 134 nm. Survival of the mice after intravenous injection in a dose of 15-20 ml/kg was 100%.

**Example 8.** PFOC emulsion with the concentration of PFD = 13.3 v% and concentration of PMCP = 6.67 v%, i.e., with the PFD:PMCP ratio of 2:1 and the total concentration of dispersion phase (PFOC) equal to 20 v%, was stabilized by proxanol 268 (Pr268) at a concentration of 3 w% and SAS-3 at a concentration of 1 w%, i.e., the ratio of SAS-3/Pr268 = 1:3 and total concentration of 4 w%. The emulsion was produced at a pressure of 20 MPa and homogenization cycle number N = 30.

The mean diameter of PFOC emulsion particles before the autoclaving was 124 nm. After sterilization (0.5 excess atmosphere for 60 min), the mean diameter of PFOC emulsion particles was 141 nm. Survival of the mice after intravenous injection in a dose of 15-20 ml/kg was 100%.

As has already been mentioned, results of the experiments are illustrated by the corresponding charts and a diagram.

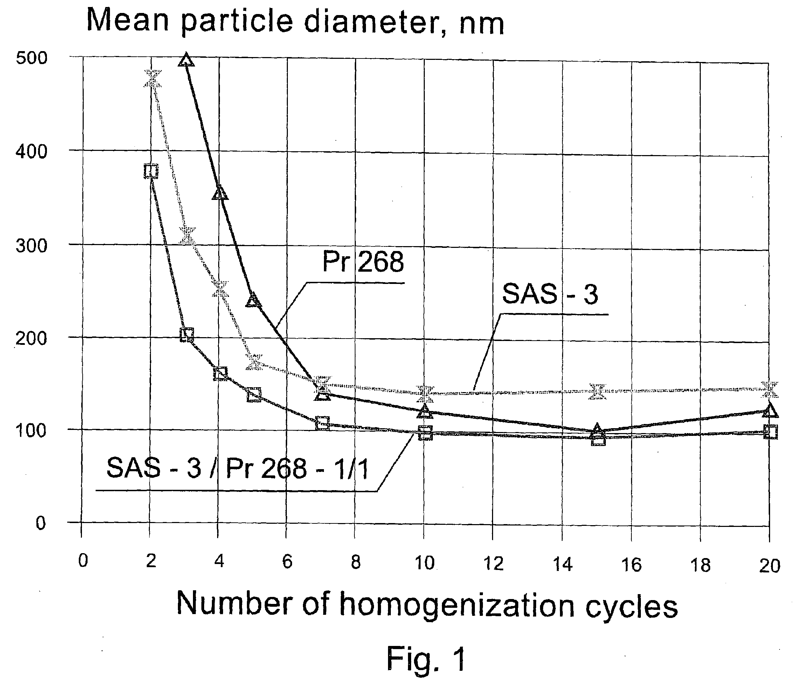

Figure 1 shows that the PFOC emulsion with SAS-3 to proxanol 268 ratio = 1:1 and total SAS concentration equal to 8 w% has the least mean particle diameter in all homogenization cycles, which leads to a conclusion that the mixture of SAS-3 and proxanol 268 has the best characteristics of changes in the mean particle diameter for the number of homogenization cycles (the dynamics of PFOC emulsification).

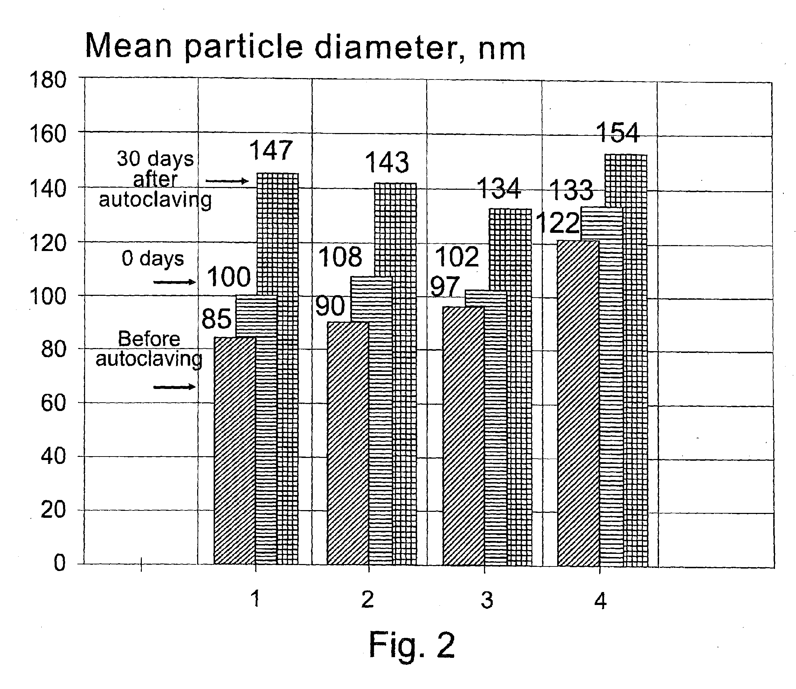

The diagram, Figure 2, leads to a conclusion that immediately after the autoclaving in all types of PFOC emulsions the mean particle diameter increases quite insignificantly. The best characteristics of mean particle diameter are typical of the PFOC emulsion with the ratio of tocopherol polyethylenglycol ester (SAS-3) to proxanol 268 = 1/3 and SAS concentration of 4 w%: 97 nm, 102 nm and 134 nm, respectively (see Example 3).

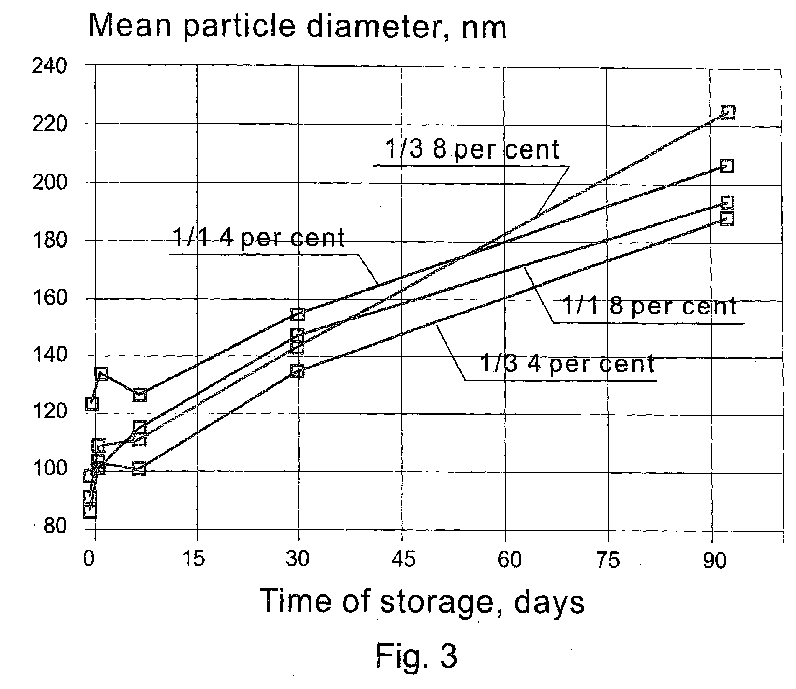

The data of Figure 3 lead to a conclusion that all emulsions are well stored at a temperature of +4-5°C, however the best results are typical of the PFOC emulsion with SAS-3 to proxanol 268 ratio of 1:3 and total SAS concentration of 4 w% (indicated as 1/3 4 per cent in Fig. 3).

Proposed PFOC emulsion with gas-transport properties, as compared with available analogs, has the following advantages:
- Stability at long-term storage;
- Possibility of storage without freezing;
- Possibility of sterilization by autoclaving;
- Absence of toxicity at intravenous injection in a dose of 15-20 ml/kg;
- Production of an emulsion with particle size of less than 150 nm at narrow dispersiveness thereof.

### Information Sources

1. Patent RF Nº 2070033, A 61 K 9/10, 10.12.1996
2. Author's certificate Nº 1298976, A 61 K 9/107, 31/02
3. Patent RF Nº 2088217, A 61 K 9/10, 31/02, 27.08.1997
4. Application EP Nº 0231070, A61K9/00, 31/02, 12.09.1989
5. Patent US Nº 5536753, A61K31/03, 17.06.1996
6. Patent US Nº 5621144, C07C223/12, 15.04.1997
7. Patent US Nº 5840767, A61K31/02, 24.11.1998
8. Kuznetsova I. N., Kruglyak Z. A. Determination of particle size of water dispersion preparation by the method of spectroturbidimetry. Chemico-Pharmaceutical Journal, 1987, 1498-1503.
9. Shchegolev S. Yu., Khlebtsev N. G. Mini- and micro-computers in determination of dispersion system parameters by spectroturbidimetric method. Manual, Saratov University Publishers, 1984.
10. Shchegolev S. Yu., Klenin V. I. Determination of the size and index of particle reflection from turbidity spectrum of dispersion systems. Optics and Spectroscopy, Moscow, 1971, v. 31, 5, 794-802.

## Claims

1. The use of tocopherol polyethylenglycol ester as a surface-active agent in production of an emulsion of perfluororganic compounds with gas-transport properties.

2. An emulsion of perfluororganic compounds with gas-transport properties, containing a mixture of perfluororganic compounds and a surface-active substance, **characterized in that** it contains a surface-active agent which is embodied at least as tocopherol polyethylenglycol ester or a mixture thereof with other nonionogenic surface-active substances.

3. Emulsion of Claim 2, **characterized in that** the ratio of the amount of perfluororganic compounds to the amount of tocopherol polyethylenglycol ester is 20:1 to 3:1 by weight.

4. Emulsion of Claim 2, **characterized in that** the ratio of the amount of perfluororganic compounds to the total amount of nonionogenic surface-active substances is 20:1 to 3:1 by weight.

5. Emulsion of Claim 2, **characterized in that** the mixture of perfluororganic compounds is a mixture of perfluordecalin and perfluoro-n-methylcyclohexylpiperidine or other perfluorated compounds, with proxanol used as an additional surface-active substance.

6. Emulsion of Claim 2, **characterized in that** the total concentration of the mixture of perfluororganic compounds in finished preparation is 5 v% to 30 v%.

7. The method of obtaining emulsion of Claim 2, where the mixture of perfluordecalin and perfluoro-n-methylcyclohexylpiperidine is stabilized by a surface-active agent, **characterized in that** tocopherol polyethylenglycol ester or a mixture thereof with other nonionogenic surface-active substances is used as a surface-active agent.

8. The method of Claim 8, **characterized in that** the above emulsion is produced at a pressure of 20-60 MPa with homogenization cycle number up to 30.

9. The method of Claim 7 **characterized in that** the above emulsion is sterilized by autoclaving in the temperature range of 100 to 120°C.
